(19) Europäisches Patentamt
European Patent Office
Office européen des brevets

(11) Veröffentlichungsnummer: **0 053 699**
**B1**

(12) # EUROPÄISCHE PATENTSCHRIFT

(45) Veröffentlichungstag der Patentschrift: **21.09.83**

(51) Int. Cl.³: **C 07 C 121/82**, A 01 N 37/44

(21) Anmeldenummer: **81108816.0**

(22) Anmeldetag: **23.10.81**

(54) **2'-Phenylhydrazino-2-cyanacrylsäureester und diese enthaltende Herbizide.**

(30) Priorität: **05.12.80 DE 3045903**
**04.07.81 DE 3126479**

(43) Veröffentlichungstag der Anmeldung: **16.06.82 Patentblatt 82/24**

(45) Bekanntmachung des Hinweises auf die Patenterteilung: **21.09.83 Patentblatt 83/38**

(84) Benannte Vertragsstaaten: **AT BE CH DE FR GB IT LI LU NL SE**

(56) Entgegenhaltungen:
**IL FARMACO - ED.SC., Band 26, (1), 1971, Seiten 66-88 Pavia, C. ALBERTI et al.: "Sulfanilammidi pirazoliche"**

**CHEMICAL ABSTRACTS, Band 67, Nr. 7, 14. August 1967, Seite 3089, Nr. 32640h Columbus, Ohio, U.S.A., CARLO ALBERTI et al.: "Sulfanilamidopyrazoles. IX. Chloro, methyl, and methoxy derivatives of 1-phenyl-5-sulfanilamidopyrazole"**

**CHEMICAL ABSTRACTS, Band 68, Nr. 3, 15. Januar 1968, Seite 1235, Nr. 12896p Columbus, Ohio, U.S.A., C. ALBERTI et al.: "Sulfanilamidopyrazoles. X. Nitro derivatives of 1-phenyl-5-sulfanilamidopyrazole and 1-phenyl-3-methyl-5-sulfanilamidopyrazole"**

(73) Patentinhaber: **BASF Aktiengesellschaft, Carl-Bosch-Strasse 38, D-6700 Ludwigshafen (DE)**

(72) Erfinder: **Eicken, Karl, Dr., Waldstrasse 63, D-6706 Wachenheim (DE)**
Erfinder: **Plath, Peter, Dr., Berner Weg 24, D-6700 Ludwigshafen (DE)**
Erfinder: **Wuerzer, Bruno, Dr., Dipl.-Landw., Ruedigerstrasse 13, D-6701 Otterstadt (DE)**

EP 0 053 699 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# 2'-Phenylhydrazino-2-cyanacrylsäureester und diese enthaltende Herbizide

Die vorliegende Erfindung betrifft 2'-Phenylhydrazino-2-cyanacrylsäureester und Herbizide, welche diese Verbindungen enthalten.

2'-Phenylhydrazino-2-cyanacrylsäureester, die ein Chlor- oder Nitroatom im Phenylrest besitzen, sind aus der Literatur bekannt (Farmaco Ed. Sci. 22 (1967), S. 58; S. 418). Sie dienen als Zwischenprodukte z.B. für die Synthese von Diuretika oder antibakterieller Verbindungen. Es ist ferner bekannt, 2'-Ethoxyphenylhydrazino-2-cyanacrylsäureethylester herzustellen (C. Alberti, C. Tironi: Sulfanilammidi pirazoliche.

il Farmaco 26 (1971) 66-88 (insbesondere S. 80). Über herbizide Eigenschaften dieser Verbindungen ist nichts bekannt.

Es wurde nun gefunden, das 2'-Phenylhydrazino-2-cyanacrylsäureester der Formel I

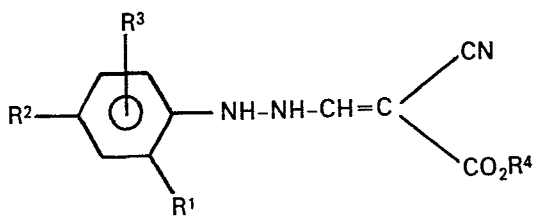

I,

in der
$R^1$ Chlor oder Brom,
$R^2$ Chlor, Brom, Jod,
$R^3$ Chlor oder Brom und
$R^4$ Alkyl mit 1 bis 3 Kohlenstoffatomen, Allyl oder Propargyl bedeutet, eine überraschend starke und gleichzeitig selektive herbizide Wirkung aufweisen.

Die 2'-Phenylhydrazino-2-cyanacrylsäureester der Formel I erhält man beispielsweise, indem man substituierte Phenylhydrazine der Formel II

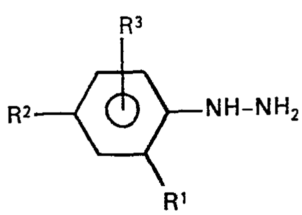

II,

mit substituierten 2-Cyanacrylsäureestern der Formel III

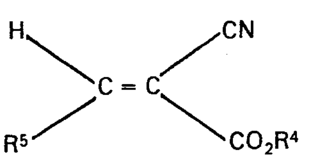

III,

wobei $R^1$, $R^2$, $R^3$, $R^4$ die oben genannten Bedeutungen haben und $R^5$ Alkoxi mit 1 bis 4 Kohlenstoffatomen oder N,N-Dialkylamino mit 1 bis 4 Kohlenstoffatomen je Alkylrest oder eine Hydroxigruppe bedeutet, umsetzt.

Hierbei ist es zweckmässig, unter schonenden Bedingungen zu arbeiten bei Temperaturen von −25 bis 70°C, vorzugsweise bei Raumtemperatur (20°C).

Als Lösungsmittel eignen sich solche, in denen beide Reaktionspartner löslich oder teilweise löslich sind, insbesondere Alkohole wie Methanol, Ethanol, Propanol, i-Propanol, Butanol, Ether wie Tetrahydrofuran, Dioxan oder auch Gemische dieser Lösungsmittel.

Die erfindungsgemässen 2'Phenylhydrazino-2-cyanacrylsäureester der Formel I kristallisieren meist aus der Reaktionslösung in reiner Form aus und können durch Absaugen und schonende Trocknung (weniger als 50°C) isoliert werden.

Die 2-Cyanacrylsäureester der Formel III werden in mindestens molarer Menge, bezogen auf die substituierten Phenylhydrazine der Formel II, eingesetzt, vorzugsweise in stöchiometrischer Menge. Werden anstelle der freien Phenylhydrazine der Formel II deren mineralsaure Salze, wie z.B. Hydrochloride oder Sulfate, eingesetzt, so ist es zweckmässig, zuerst das substituierte Phenylhydrazin der Formel II durch Zugabe einer äquivalenten Menge an Alkalialkoholat oder Alkaliacetat freizusetzen und dann die Umsetzung auszuführen.

Die verwendeten Phenylhydrazine der Formel II sind bekannt oder können nach bekannten Methoden hergestellt werden (Methoden der Organ. Chemie, Houben-Weyl, Bd. 10/2, S. 180 ff). Die eingesetzten 2-Cyanacrylsäureester der Formel III sind bekannt oder können nach bekannten Methoden hergestellt werden (DOS 2 635 841; Chem. Ber. (1964) 97, 3397).

In den folgenden Beispielen verhalten sich Gewichtsteile zu Volumenteilen wie Kilogramm zu Liter.

Herstellung der 2'-Phenylhydrazino-2-cyanacrylsäureester der Formel I:

Beispiel 1

148,1 Gewichtsteile 2,4,6-Trichlorphenylhydrazin werden in eine Lösung von 108,5 Gewichtsteilen Ethoximethylen-2-cyanessigsäuremethylester in 1000 Volumenteilen Methanol gegeben. Aus der Lösung fällt ein Kristallbrei aus, der nach 3-stündigem Rühren, Absaugen und Trocknen im Vakuum bei 40°C 187,4 Gewichtsteile 2'-(2,4,6-Trichlorphenyl)-hydrazino-2-cyanacrylsäuremethylester vom Fp. 174 bis 175°C liefert (Verbindung Nr. 1).

$C_{11}H_8Cl_3N_3O_2$ (M 320,5)
Ber.: C 41,22 H 2,52 N 13,11
Gef.: C 40,9 H 2,8 N 12,8

In entsprechender Weise können folgende 2'-Phenylhydrazino-2-cyanacrylsäureester der Formel I hergestellt werden:

| Nr. | $R^1$ | $R^2$ | $R^3$ | $R^4$ | Fp (°C) |
|---|---|---|---|---|---|
| 2 | Cl | Cl | 6-Cl | $C_2H_5$ | 166 |
| 3 | Cl | Cl | 6-Cl | i-$C_3H_7$ | 130 |
| 4 | Br | Br | 6-Br | $CH_3$ | 182 |
| 5 | Cl | Br | 5-Cl | $CH_3$ | |
| 6 | Cl | Cl | 5-Cl | $CH_3$ | 195 |
| 7 | Cl | Cl | 6-Br | $CH_3$ | |
| 8 | Cl | Br | 6-Br | $CH_3$ | |
| 9 | Cl | Br | 6-Cl | $CH_3$ | |
| 10 | Br | Cl | 6-Br | $CH_3$ | |
| 11 | Cl | Cl | 6-Cl | $CH_2CH=CH_2$ | 156 |
| 12 | Cl | Cl | 6-Cl | Propargyl | |

Die Anwendung als Herbizid erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Vesprühen, Streichen, Tränken, Vernebeln, Verstäuben, Verstreuen oder Giessen. Die Anwendungsformen richten sich ganz nach den Verwendungszwecken; sie sollen in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw. sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Ethanol, Propanol, Butanol, Chloroform, Tetrachlorkohlenstoff, Cyclohexanol, Cyclohexanon, Chlorbenzol, Isophoran usw., stark polare Lösungsmittel, z.B. Dimethylformamid, Dimethylsulfoxid, N-Methylpyrrolidon, Wasser usw. in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Herbizide enthalten z.B. 5 bis 95% (Gew.-%) insbesondere 10 bis 80% Wirkstoff.

An oberflächenaktiven Stoffen sind zu nennen: Alkali-, Erdalkali-, Ammoniumsalze von Ligninsulfonsäure, Napthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Napthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether. Tributylphenylpolyglykolether, Alkylarylpolyetheralkoholate, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Kalk, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Beispiel a

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel b

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

Beispiel c

20 Gewichtsteile der Verbindung 2 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

Beispiel d

20 Gewichtsteile der Verbindung 1 werden in einer Mischung gelöst, die aus 25 Gewichtsteilen Cyclohexanon, 65 Gewichtsteilen einer Mineralölfraktion vom Siedepunkt 210 bis 280°C und 10

Gewichtsteilen des Anlagerungsproduktes von 40 Mol Ethylenoxid an 1 Mol Ricinusöl besteht.

Beispiel e
80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel f
5 Gewichtsteile der Verbindung 2 werden mit 95 Gewichtsteilen feinteiligem Kaolin innig vermischt. Man erhält auf diese Weise ein Stäubemittel, das 5 Gewichtsprozent des Wirkstoffs enthält.

Beispiel g
30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel h
40 Gewichtsteile des Wirkstoffs 2 werden mit 10 Teilen Natriumsalz eines Phenolsulfonsäureharnstoff-formaldehyd-kondensats, 2 Teilen Kieselgel und 48 Teilen Wasser innig vermischt. Man erhält eine stabile wässrige Dispersion. Durch Verdünnen mit Wasser erhält man eine wässrige Dispersion.

Beispiel i
20 Teile des Wirkstoffs 1 werden mit 12 Teilen Calciumsalz der Dodecylbenzolsulfonsäure, 8 Teile Fettalkohol-polyglykolether, 2 Teilen Natriumsalz eines Phenolsulfonsäureharnstoff-formaldehyd-kondensats und 68 Teilen eines paraffinischen Mineralöls innig vermischt. Man erhält eine stabile ölige Dispersion.

Der Einfluss von Vertretern der neuen 2′-Phenylhydrazino-2-cyanacrylsäureester auf das Wachstum von erwünschten und unerwünschten Pflanzen wird anhand von Gewächshausversuchen vorgeführt.

Als Kulturgefässe dienten Plastikblumentöpfe mit 300 $cm^3$ Inhalt und lehmiger Sand mit etwa 1,5% Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Wirkstoff/ha. Kurz vor oder nach dem Aufbringen der Mittel wurden die Gefässe leicht beregnet, um Keimung und Wachstum in Gang zu bringen. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 3 bis 10 cm an und behandelte sie danach.

Zur Nachauflaufbehandlung wurden entweder direkt gesäte und in den gleichen Gefässen aufgewachsene Pflanzen ausgewählt, oder sie wurden erst als Keimpflanzen getrennt angezogen und einige Tage vor der Behandlung in die Versuchsgefässe verpflanzt. Es ist zu erwähnen, dass bei dem für das Nachauflaufverfahren benutzen Reis das Substrat mit Torfmull (peat) angereichert war. Dasselbe gilt für Klettenlabkraut. Die Aufwandmenge für die Nachauflaufbehandlung betrug 0,5 kg/ha und 3,0 kg/ha Wirkstoff für die neue Verbindung Nr. 1.

Allgemein unterblieb bei der Nachauflaufbehandlung die Abdeckung. Die Aufstellung der Versuche erfolgte im Gewächshaus, wobei für wärmeliebende Arten wärmere Bereiche (20 bis 35°C) und für solche gemässigter Klimate 15 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die Gewächshausversuche ergeben, dass der Wirkstoff Nr. 1 mit 3,0 kg Wirkstoff/ha bei Vor- und Nachauflaufanwendung eine gute herbizide Wirkung hat.

In diesen Gewächshausversuchen hat die Verbindung Nr. 1 mit 0,5 kg Wirkstoff/ha zudem eine sehr gute Wirkung gegen eine ganze Reihe von unerwünschten Pflanzen. Gewisse Kulturpflanzen tolerieren die Behandlung mit diesem Wirkstoff bei höchstenfalls geringfügiger temporärer Schädigung.

Sind Kulturpflanzen bei Blattbehandlung gegenüber den erfindungsgemässen Wirkstoffen etwas empfindlicher, so können auch Ausbringungstechniken angewandt werden, bei welchen die herbiziden Mittel mit Hilfe der Spritzgeräte so geleitet werden, dass die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by). In Anbetracht der Vielseitigkeit der Applikationsmethode können die erfindungsgemässen Herbizide noch in einer weiteren grossen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

Zur Verbreiterung des Wirkungsspektrums und zur Erzielung synergistischer Effekte können die neuen erfindungsgemässen Verbindungen mit

zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dionderivate und andere in Betracht.

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Tabelle 1 – Liste der Pflanzennamen

| Botanischer Name | Deutscher Name | Englischer Name |
|---|---|---|
| Abutilon theophrasti | Chinesischer Hanf | velvet leaf |
| Amaranthus spp. | zurückgekrümmte Fuchsschwanzarten | pigweed |
| Centaurea cyanus | Kornblume | cornflower |
| Chenopodium album | Weisser Gänsefuss | lambsquarters |
| Daucus carota | Wilde Möhre | wild carrot |
| Galium aparine | Klettenlabkraut | catchweed bedstraw |
| Ipomoea spp. | Prunkwindearten | morningglory |
| Lamium purpureum | rote Taubnessel | henbit |
| Oryza sativa | Reis | rice |
| Sida spinosa | – | teaweed (prickly sida) |
| Sinapis alba | Weisser Senf | white mustard |
| Solanum nigrum | Schwarzer Nachtschatten | black nightshade |
| Triticum aestivum | Weizen | wheat |

**Patentansprüche** für die Vertragsstaaten:
BE, CH, DE, FR, GB, IT, LI, LU, NL, SE

1. 2'-Phenylhydrazino-2-cyanacrylsäureester der Formel

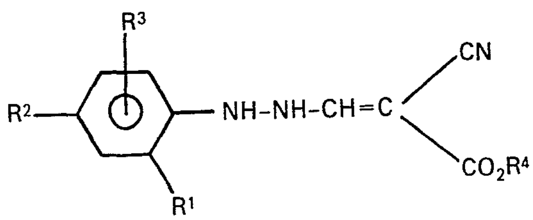

I,

in der
$R^1$ Chlor oder Brom,
$R^2$ Chlor, Brom, Jod,
$R^3$ Chlor oder Brom und
$R^4$ Alkyl mit 1 bis 3 Kohlenstoffatomen, Allyl oder Propargyl bedeutet.

2. Herbizid, enthaltend ein 2'-Phenylhydrazino-2-cyanacrylsäureester gemäss Anspruch 1.

3. Herbizid, enthaltend einen festen oder flüssigen Trägerstoff und einen 2'-Phenylhydrazino-2-cyanacrylsäureester gemäss Anspruch 1.

4. Verfahren zur Herstellung eines Herbizids, dadurch gekennzeichnet, dass man einen festen oder flüssigen Trägerstoff vermischt mit einem 2'-Phenylhydrazino-2-cyanacrylsäureester gemäss Anspruch 1.

5. Verfahren zur Bekämpfung von unerwünschten Pflanzen, dadurch gekennzeichnet, dass man die Pflanzen oder den Boden behandelt mit einem 2'-Phenylhydrazino-2-cyanacrylsäureester gemäss Anspruch 1.

6. Verfahren zur Herstellung eines 2'-Phenylhydrazino-2-cyanacrylsäureesters gemäss Anspruch 1, dadurch gekennzeichnet, dass man ein substituiertes Phenylhydrazin der Formel

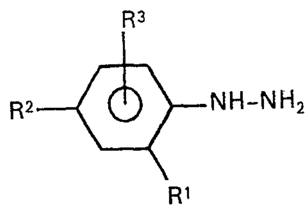

II,

in der $R^1$, $R^2$ und $R^3$ die im Anspruch 1 genannten Bedeutungen haben, oder dessen mineralsaure Salze mit einem substituierten 2-Cyanacrylsäureester der Formel

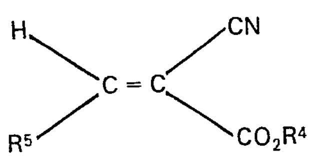

III,

in der $R^4$ die im Anspruch 1 angegebene Bedeutung hat und $R^5$ Alkoxi mit 1 bis 4 Kohlenstoffatomen oder N,N-Dialkylamino mit 1 bis zu 4 Kohlenstoffatomen je Alkylgruppe oder eine Hydroxigruppe bedeutet, gegebenenfalls in Gegenwart eines säurebindenden Mittels, umsetzt.

7. 2'-(2,4,6-Trichlorphenyl)-hydrazino-2-cyanacrylsäuremethylester.

8. Herbizid, enthaltend 2'-(2,4,6-Trichlorphenyl)-hydrazino-2-cyanacrylsäuremethylester.

**Patentansprüche** für den Vertragsstaat: A–

1. Herbizid, enthaltend ein 2'-Phenylhydrazino-2-cyanacrylsäureester der Formel

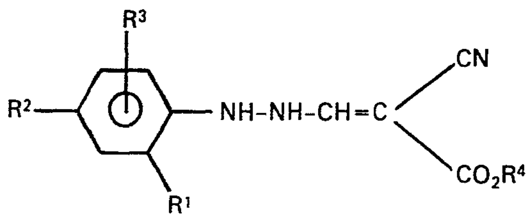

I,

in der
$R^1$ Chlor oder Brom,
$R^2$ Chlor, Brom, Jod,
$R^3$ Chlor oder Brom und
$R^4$ Alkyl mit 1 bis 3 Kohlenstoffatomen, Allyl oder Propargyl bedeutet.

2. Herbizid, enthaltend einen festen oder flüssigen Trägerstoff und ein 2'-Phenylhydrazino-2-cyanacrylsäureester gemäss Anspruch 1.

3. Verfahren zur Herstellung eines Herbizids, dadurch gekennzeichnet, dass man einen festen oder flüssigen Trägerstoff vermischt mit einem 2'-Phenylhydrazino-2-cyanacrylsäureester gemäss Anspruch 1.

4. Verfahren zur Bekämpfung von unerwünschten Pflanzen, dadurch gekennzeichnet, dass man die Pflanzen oder den Boden behandelt mit einem 2'-Phenylhydrazino-2-cyanacrylsäureester gemäss Anspruch 1.

5. Verfahren zur Herstellung eines 2'-Phenylhydrazino-2-cyanacrylsäureesters gemäss Anspruch 1, dadurch gekennzeichnet, dass man ein substituiertes Phenylhydrazin der Formel

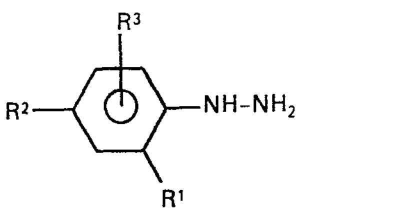

II,

in der $R^1$, $R^2$, und $R^3$ die im Anspruch 1 genannten Bedeutungen haben, oder dessen mineralsaure Salze mit einem substituierten 2-Cyanacrylsäureester der Formel

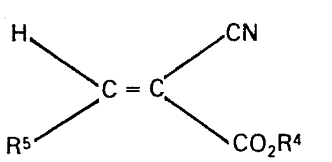

III,

in der $R^4$ die im Anspruch 1 angegebene Bedeutung hat und $R^5$ Alkoxi mit 1 bis 4 Kohlenstoffatomen oder N,N-Di-alkylamino mit 1 bis zu 4 Kohlenstoffatomen je Alkylgruppe oder eine Hydroxigruppe bedeutet, gegebenenfalls in Gegenwart eines säurebindenden Mittels, umsetzt.

6. Herbizid, enthaltend 2'-(2,4,6-Trichlorphenyl)-hydrazino-2-cyanacrylsäuremethylester.

**Revendications** pour l'Etat contractant: AT

1. Herbicide contenant un ester d'acide 2'-phénylhydrazino-2-cyanacrylique de formule

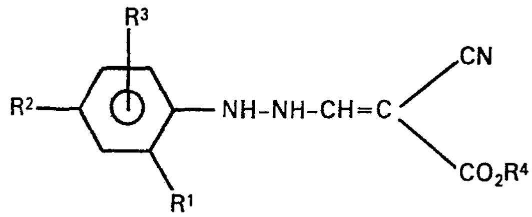

I,

dans laquelle
$R^1$ représente chlore ou brome
$R^2$ chlore, brome, iode
$R^3$ chlore ou brome et
$R^4$ alkyle à 1 à 3 atomes de carbone, allyle ou propargyle.

2. Herbicide contenant un support solide ou liquide et un ester d'acide 2'-phénylhydrazino-2-cyanacrylique selon la revendication 1.

3. Procédé de préparation d'un herbicide, caractérisé par le fait qu'on mélange un support solide ou liquide avec un ester d'acide 2'-phénylhydrazino-2-cyanacrylique selon la revendication 1.

4. Procédé de lutte contre les plantes indésirables caractérisé par le fait qu'on traite les plantes ou le sol avec un ester d'acide 2'-phénylhydrazino-2-cyanacrylique selon la revendication 1.

5. Procédé de préparation d'un ester d'acide 2'-phénylhydrazino-2-cyanacrylique selon la revendication 1, caractérisé par le fait qu'on fait réagir une phénylhydrazine substituée de formule

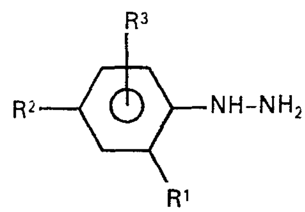

II,

dans laquelle $R^1$, $R^2$ et $R^3$ ont les significations indiquées dans la revendication 1, ou leurs sels d'acide minéral, avec un ester d'acide 2-cyanacrylique substitué, de formule

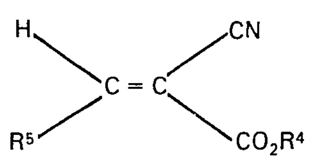

III,

dans laquelle $R^4$ a la signification donnée dans la revendication 1 et $R^5$ représente alcoxy à 1 à 4 atomes de carbone ou N,N-dialkylamino à 1 à 4 atomes de carbone par groupe alkyle ou un groupe hydroxy, éventuellement en présence d'un agent liant l'acide.

6. Herbicide contenant le 2'-(2,4,6-trichlorophényl)-hydrazino-2-cyanacrylate de méthyle.

**Revendictions** pour les Etats contractants: BE, CH, DE, FR, GB, IT, LI, LU, NL, SE

1. Ester d'acide 2'-phénylhydrazino-2-cyanacrylique de formule

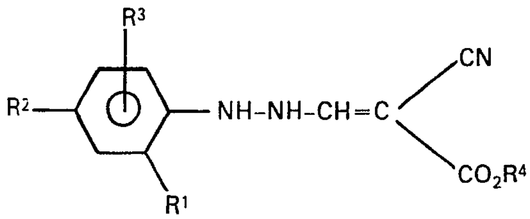

I,

dans laquelle
$R^1$ représente chlore ou brome
$R^2$ chlore, brome, iode
$R^3$ chlore ou brome et
$R^4$ alkyle à 1 à 3 atomes de carbone, allyle ou propargyle.

2. Herbicide, contenant un ester d'acide 2'-phénylhydrazino-2-cyanacrylique selon la revendication 1.

3. Herbicide contenant un support solide ou liquide et un ester d'acide 2'-phénylhydrazino-2-cyanacrylique selon la revendication 1.

4. Procédé de préparation d'un herbicide, caractérisé par le fait qu'on mélange un support solide ou liquide avec un ester d'acide 2'-phénylhydrazino-2-cyanacrylique selon la revendication 1.

5. Procédé de lutte contre les plantes indésirables caractérisé par le fait qu'on traite les plantes ou le sol avec un ester d'acide 2'-phénylhydrazino-2-cyanacrylique selon la revendication 1.

6. Procédé de préparation d'un ester d'acide 2'-phénylhydrazino-2-cyanacrylique selon la revendication 1, caractérisé par le fait qu'on fait réagir une phénylhydrazine substituée de formule

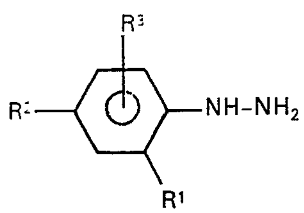

II,

dans laquelle $R^1$, $R^2$ et $R^3$ ont les significations indiquées dans la revendication 1, ou leurs sels d'acide minéral, avec un ester d'acide 2-cyanacrylique substitué, de formule

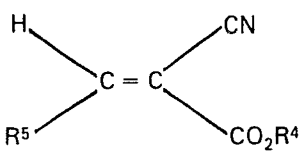

III,

dans laquelle $R^4$ a la signification donnée dans la revendication 1 et $R^5$ représente alcoxy à 1 à 4 atomes de carbone ou N,N-dialkylamino à 1 à 4 atomes de carbone par groupe alkyle ou un groupe hydroxy, éventuellement en présence d'un agent liant l'acide.

7. 2'-(2,4,6-trichlorophényl)-hydrazino-2-cyanacrylate de méthyle.

8. Herbicide contenant le 2'-(2,4,6-trichlorophényl)-hydrazino-2-cyanacrylate de méthyle.

**Claims** for the Contracting state: AT

1. A herbicide containing a 2'-phenylhydrazino-2-cyanoacrylic acid ester of the formula

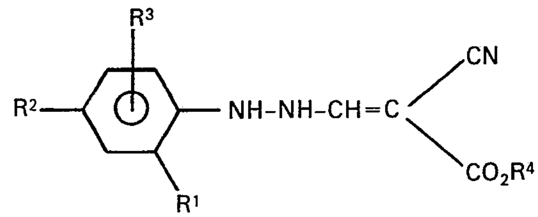

I,

where $R^1$ is chlorine or bromine, $R^2$ is chlorine, bromine or iodine, $R^3$ is chlorine or bromine, and $R^4$ is alkyl of 1 to 3 carbon atoms, allyl or propargyl.

2. A herbicide containing a solid or liquid carrier and a 2'-phenylhydrazino-2-cyanoacrylic acid ester as claimed in claim 1.

3. A process for producing a herbicide, wherein a solid or liquid carrier is mixed with a 2'-phenylhydrazino-2-cyanoacrylic acid ester as claimed in claim 1.

4. A process for combating unwanted plants, wherein the plants or the soil are treated with a 2'-phenylhydrazino-2-cyanoacrylic acid ester as claimed in claim 1.

5. A process for the manufacture of a 2'-phenylhydrazino-2-cyanoacrylic acid ester as claimed in claim 1, wherein a substituted phenylhydrazine of the formula

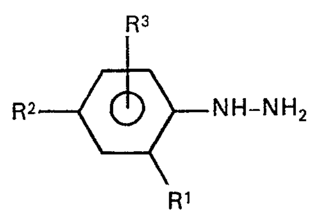

II,

where $R^1$, $R^2$ and $R^3$ have the meanings given in claim 1, or a mineral acid salt thereof, is reacted – is desired, in the presence of an acid binder – with a substituted 2-cyanoacrylic acid ester of the formula

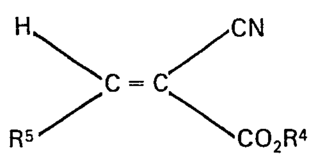

III,

where $R^4$ has the meaning given in claim 1 and $R^5$ is alkoxy of 1 to 4 carbon atoms, N,N-dialkylamino, where alkyl is of 1 to 4 carbon atoms, or hydroxyl.

6. A herbicide containing methyl 2′-(2,4,6-trichlorophenyl)-hydrazino-2-cyanoacrylate.

**Claims** for the Contracting states: BE, CH, DE, FR, GB, IT, LI, LU, NL, SE

1. A 2′-phenylhydrazino-2-cyanoacrylic acid ester of the formula

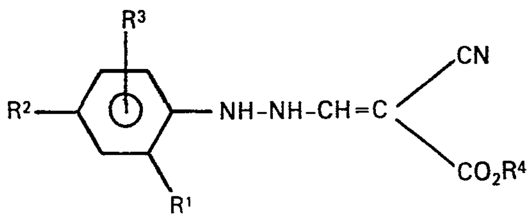

I,

where $R^1$ is chlorine or bromine, $R^2$ is chlorine, bromine or iodine, $R^3$ is chlorine or bromine, and $R^4$ is alkyl of 1 to 3 carbon atoms, allyl or propargyl.

2. A herbicide containing a 2′-phenylhydrazino-2-cyanoacrylic acid ester as claimed in claim 1.

3. A herbicide containing a solid or liquid carrier and a 2′-phenylhydrazino-2-cyanoacrylic acid ester as claimed in claim 1.

4. A process for producing a herbicide, wherein a solid or liquid carrier is mixed with a 2′-phenylhydrazino-2-cyanoacrylic acid ester as claimed in claim 1.

5. A process for combating unwanted plants, wherein the plants or the soil are treated with a 2′-phenylhydrazino-2-cyanoacrylic acid ester as claimed in claim 1.

6. A process for the manufacture of a 2′-phenylhydrazino-2-cyanoacrylic acid ester as claimed in claim 1, wherein a substituted phenylhydrazine of the formula

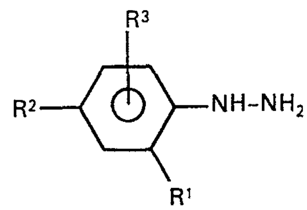

II,

where $R^1$, $R^2$ and $R^3$ have the meanings given in claim 1, or a mineral acid salt thereof, is reacted – if desired, in the presence of an acid binder – with a substituted 2-cyanoacrylic acid ester of the formula

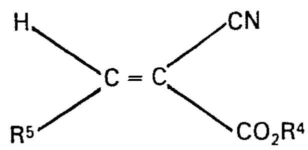

III,

where $R^4$ has the meaning given in claim 1 and $R^5$ is alkoxy of 1 to 4 carbon atoms, N,N-dialkylamino, where alkyl is of 1 to 4 carbon atoms, or hydroxyl.

7. Methyl 2′-(2,4,6-trichlorophenyl)-hydrazino-2-cyanoacrylate.

8. A herbicide containing methyl 2′-(2,4,6-trichlorophenyl)-hydrazino-2-cyanoacrylate.